(19) Europäisches Patentamt / European Patent Office / Office européen des brevets

(11) **EP 3 388 128 B1**

(12) **FASCICULE DE BREVET EUROPEEN**

(45) Date de publication et mention de la délivrance du brevet:
**07.06.2023 Bulletin 2023/23**

(21) Numéro de dépôt: **18165968.1**

(22) Date de dépôt: **05.04.2018**

(51) Classification Internationale des Brevets (IPC):
**B01D 15/18** *(2006.01)* **B01D 15/22** *(2006.01)*
**C07C 7/12** *(2006.01)* **G01N 30/60** *(2006.01)*

(52) Classification Coopérative des Brevets (CPC):
(C-Sets disponibles)
**B01D 15/185; B01D 15/1842; B01D 15/22; C07C 7/12; G01N 30/6017** (Cont.)

(54) **NOUVEAU DESSIN DES CANAUX DE COLLECTE ET DE DISTRIBUTION POUR UN PROCEDE DE SEPARATION EN LIT MOBILE SIMULE UTILISANT N-COLONNES EN SERIE**

NEUES DESIGN VON SAMMEL- UND VERTEILKANÄLEN FÜR EIN SIMULIERTES FLIESSBETT-ABSCHEIDUNGSVERFAHREN, DAS N-SÄULEN IN REIHE VERWENDET

NEW PATTERN OF COLLECTION AND DISTRIBUTION CHANNELS FOR A SIMULATED MOVING BED SEPARATION METHOD USING N COLUMNS IN SERIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.04.2017 FR 1753216**

(43) Date de publication de la demande:
**17.10.2018 Bulletin 2018/42**

(73) Titulaire: **IFP Energies nouvelles**
**92852 Rueil-Malmaison Cedex (FR)**

(72) Inventeurs:
• **AUGIER, Frederic**
  **69360 SAINT SYMPHORIEN D OZON (FR)**
• **ROYON-LEBEAUD, Aude**
  **69007 LYON (FR)**
• **LEINEKUGEL LE COCQ, Damien**
  **69600 OULLINS (FR)**

• **VONNER, Alexandre**
  **69780 MIONS (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
EP-A2- 2 138 839    WO-A1-00/50144
WO-A1-99/62609    WO-A1-2011/159232
US-A1- 2004 140 007    US-B1- 6 171 502

**EP 3 388 128 B1**

(52) Classification Coopérative des Brevets (CPC):
(Cont.)

C-Sets
**C07C 7/12, C07C 15/02**

**Description**

CONTEXTE DE L'INVENTION

L'invention concerne un nouveau dispositif de distribution et de collecte de fluides au sein d'un système à N-colonnes en série mettant en oeuvre un écoulement desdits fluides dans un milieu de particules solides, appelé milieu granulaire.

On appelle système à N-colonnes en série un enchainement de N colonnes distinctes ou empilées (c'est-à-dire arrangées dans une enveloppe commune). Chaque colonne abrite un lit du milieu granulaire dans lequel s'écoule le fluide principal. Les N colonnes sont connectées entre elles par un réseau de conduites permettant éventuellement l'injection/mélange ou le soutirage d'un fluide secondaire, ces injections et soutirages possibles ayant lieu entre les colonnes.

La présente invention concerne le dispositif qui permet de collecter en fond de lit ou sur la section complète de la colonne le fluide principal vers, réciproquement, depuis la conduite inter-colonne qui permet de transférer ledit fluide vers la colonne suivante.

L'invention consiste essentiellement en un aménagement de la zone de collecte ou de distribution située en fond de colonne.

L'invention permet de compenser de façon avantageuse la distribution de temps de séjour du fluide dans une colonne entre les deux zones dites non sélectives, c'est-à-dire en dehors du milieu granulaire, que constituent la zone de distribution en tête de lit et la zone de collecte en fond de lit, tout en minimisant fortement les volumes des zones dites non sélectives.

En effet, l'invention concerne les procédés de séparation en Lit Mobile Simulé (LMS) pour lesquels la synchronicité de l'ensemble des trajectoires fluides au sein du lit, dans le système de distribution, et au sein du réseau d'injection / collecte est particulièrement critique pour garantir les hauts niveaux de pureté et de rendement requis par le procédé. On parle dans la suite de manière abrégée de synchronicité du fluide.

On entend par synchronicité du fluide le fait que si l'on divise par la pensée la section de la colonne en une multiplicité de canaux fluides évoluant en parallèle, tous les canaux fluides doivent autant que possible avoir le même temps de séjour depuis l'entrée dans la colonne jusqu'à la sortie.

Par ailleurs, toute augmentation des volumes des zones non sélectives induit une augmentation non désirée des débits « de tourne en rond » (traduction de la terminologie anglo saxonne « pump around ») dans l'installation, à mêmes niveaux de performance, ce qui est néfaste à la sélectivité du procédé ou augmente la consommation énergétique du procédé dans son ensemble

DESCRIPTION SOMMAIRE DES FIGURES

L'ensemble des figures est représenté pour un écoulement descendant, mais l'invention est strictement symétrique pour un écoulement ascendant.

La figure 1 représente une vue schématique de l'ensemble de la colonne avec son dispositif de distribution des fluides en tête et son dispositif de collecte en fond.
La figure 1a est une vue de dessus qui permet de visualiser l'emplacement du réseau de collecte en périphérie.
La figure 2 représente une vue schématique de la colonne dans une variante dite en écoulement radial du fluide avec le réseau de collecte selon l'invention.

EXAMEN DE L'ART ANTERIEUR

L'art antérieur enseigne comment dimensionner un distributeur pour une colonne multi étagée c'est-à-dire constituée d'une multiplicité de plateaux disposés selon un axe sensiblement vertical, chaque plateau supportant un lit de solide granulaire.

Les brevets EP0074815, US2006/0108274A1, en particulier, fournissent des exemples de dispositifs de distribution/mélanges utilisés dans le cas de l'adsorption en lit mobile simulé pour une colonne multi-étagée. Ces dispositifs assurent de façon successive les fonctions de collecte du fluide principal en provenance du lit amont, mélange du fluide principal avec le fluide secondaire, et redistribution du mélange vers le lit aval.

Ces brevets décrivent la division de la section de la colonne en panneaux ou secteurs radiaux.

L'art antérieur enseigne également l'importance de garantir une bonne synchronicité dans le système de distribution pour obtenir les performances requises pour la séparation.

En particulier le brevet FR2933000 propose d'ajouter un élément de compensation du temps de séjour, une chicane, dans la zone non sélective juste avant ou juste après le plateau distributeur en entrée de colonne.

Le document de l'art antérieur de Silva M., Rodrigues A. et Mota J. « Effect of dead volumes on the performance of an industrial-scale simulated moving-bed parex unit or p-xylene purification », paru dans l'AIChE Journal janv. 2016, vol 62, n°1, (et qu'on peut traduire par « effets des volumes morts sur les performances d'une unité Parex en lit mobile simulé »), enseigne l'importance de diminuer les volumes non sélectifs car l'augmentation de ces volumes entraine une baisse de pureté et une augmentation de la consommation de désorbant.

Le brevet US3,214,247 décrit une chambre de contact destinée à alimenter un lit granulaire, ladite chambre présentant des perforations latérales qui permettent d'introduire la phase gaz ou liquide latéralement dans deux directions opposées. On voit sur la figure 3 de ce texte un canal de forme conique à paroi rectiligne.

Le brevet US3,789,989 décrit un équipement pour la distribution de fluides pour permettre le passage dudit fluide d'un lit au lit suivant. L'équipement est caractérisé par des écrans pour retenir le solide et des plaques de déflexion situées entre les écrans et ayant une épaisseur décroissante. Aucun de ces systèmes ne présente de dispositif en vue d'une compensation des temps de séjour.

La demande de brevet WO 99/62609 A1 décrit des distributeurs de flux comprenant deux sections ou plus dans lesquelles au moins une des sections, si elle est fournie seule et alimentée par un flux volumétrique uniforme, fournirait une distribution de débit volumétrique non uniforme à travers la zone d'écoulement et/ou des temps de séjour de ligne de courant non uniformes. Cependant, lorsque deux ou plusieurs de ces sections sont fournies en combinaison, elles ont pour résultat un distributeur d'écoulement qui fournit un débit volumétrique uniforme à travers la zone d'écoulement et des temps de séjour de ligne de courant uniformes.

Le brevet US 6,171,502 B1 décrit un embout de colonne pour une colonne de chromatographie. L'embout de colonne comprend une partie de base, une pièce d'extension pour un tube à décharge et un fritté. Le fritté produit une chute de pression par unité de longueur qui est au moins deux fois supérieure à la chute de pression par unité de longueur créée dans le garnissage de sorbant lorsque l'éluant s'écoule à travers.

La mise en oeuvre d'un lit mobile simulé par un système à N-colonnes permet d'assurer les injections et collectes, ainsi que les mélanges en un seul point entre deux colonnes dans le réseau. Une telle configuration permet de s'affranchir des problèmes de rinçage car elle minimise ainsi les volumes non rincés par le débit de tourne en rond .La présente invention introduit en plus de cet avantage un dispositif dont la forme géométrique permet de garantir une quasi égalité des temps de séjour du fluide, quel que soit le trajet emprunté par ce dernier pour traverser le lit granulaire, tout en minimisant le temps de séjour dans les zones dites non sélectives.

## DESCRIPTION SOMMAIRE DE L'INVENTION

La présente invention peut se définir comme un dispositif de distribution et de collecte du fluide dans une colonne comportant un lit de solide granulaire, l'unité étant en général composée de plusieurs colonnes disposées en série

Ce dispositif est particulièrement adapté au procédé de séparation en lit mobile simulé, car il permet une réduction substantielle des volumes dit non sélectifs en minimisant fortement la dispersion de ces zones, comme cela est illustré dans l'exemple faisant partie de la présente demande.

Dans la suite on parle de fluide entrant pour désigner le fluide entrant dans la colonne et de fluide sortant pour désigner le fluide sortant de la colonne.

Le procédé utilisant le présent dispositif fait généralement appel à plusieurs colonnes en série, chaque colonne utilisant le dispositif selon l'invention soit comme moyen d'introduction du fluide entrant, soit comme moyen de collecte du fluide sortant.

Ces colonnes peuvent être à écoulement axial ou radial du fluide à l'intérieur du lit granulaire. Toute disposition de la série de colonnes munies du dispositif selon l'invention, soit en moyen d'introduction, soit en moyen de collecte rentre dans le cadre de la présente invention.

De façon plus précise, la présente invention peut donc se définir comme un dispositif de distribution de fluide ou de collecte du fluide sortant dans une ou des colonnes muni(es) d'un lit de solide granulaire tel que défini dans la revendication 1, dans lequel :

- la distribution du fluide entrant se fait au moyen d'un canal de distribution de section variable dans lequel le fluide s'écoule à une vitesse constante $V_{canal}$ comprise entre 0,1 et 5 m/s,

- la collecte du fluide sortant se fait au moyen d'un canal de collecte de section variable dans lequel le fluide s'écoule à vitesse constante.

Le présent dispositif s'applique particulièrement à un procédé de séparation en lit mobile simulé utilisant une pluralité de colonnes disposées en série.

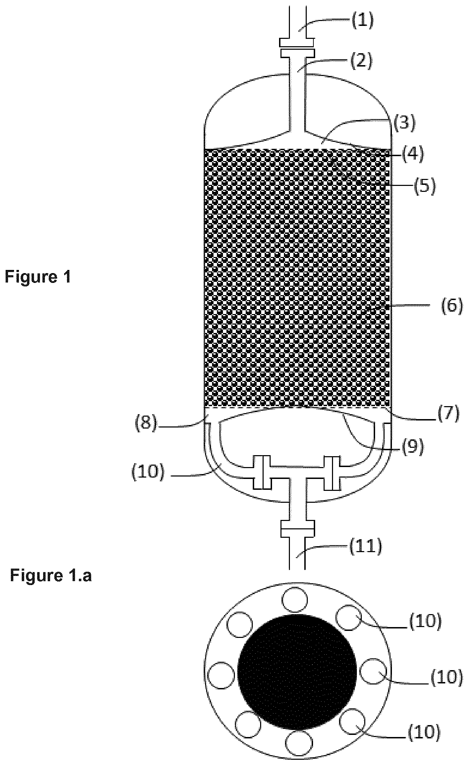

Dans ce cas, et selon une première variante, présentée sur les figures 1 et 1.a, le procédé en lit mobile simulé utilisant le dispositif de collecte selon l'invention peut se décrire de la façon suivante :

La circulation du fluide à l'intérieur du lit de solide granulaire est axiale, et l'injection dans le lit se fait au moyen d'une conduite (2) sensiblement centrée sur l'axe vertical de la colonne, qui alimente le canal de distribution horizontal (3) délimité par une paroi incurvée (4), le lit de solide granulaire (6) étant ensuite alimenté depuis ledit canal de distribution (3) au travers d'une grille (5), et le fluide s'écoule au travers du lit granulaire (6) selon une direction sensiblement verticale, le fluide étant ensuite collecté sous la grille (7) à partir du canal de collecte (8) délimité par une paroi incurvée (9) par l'intermédiaire des conduites périphériques (10), et l'ensemble du débit étant rassemblé dans une conduite d'évacuation unique (11) sensiblement centrée selon l'axe vertical de la colonne.

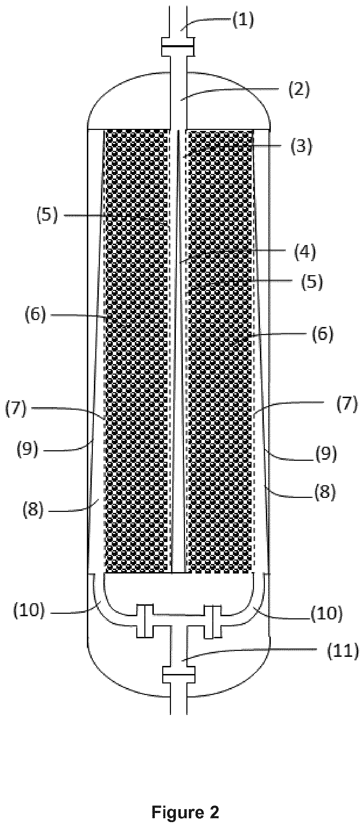

Selon une seconde variante, présentée sur la figure 2, le procédé utilisant le dispositif de distribution selon l'invention peut se décrire de la façon suivante :

La circulation des fluides à l'intérieur du lit de solide granulaire se fait de façon radiale, le fluide étant introduit au centre de la colonne par la conduite (2) qui alimente le canal central (3) délimité au centre par la paroi (4), puis traversant le lit granulaire (6) depuis le centre vers la périphérie (délimité par les grilles (5) et (7)) où il est collecté dans la zone périphérique (8) définie à l'extérieur par la paroi (9), qui ramène le liquide par l'intermédiaire des conduites périphériques (10) vers la conduite d'évacuation (11) sensiblement centrée selon l'axe de la colonne.

Le procédé de séparation en lit mobile simulé faisant appel au dispositif selon l'invention peut être tel que la charge à séparer est un mélange quelconque de composés aromatiques ayant de 7 à 9 atomes de carbone.

Le procédé de séparation en lit mobile simulé faisant appel au dispositif selon l'invention peut être tel que la charge à séparer est un mélange de normales et d'iso paraffines.

Le procédé de séparation en lit mobile simulé faisant appel au dispositif selon l'invention peut être tel que la charge à séparer est un mélange de normales et d'iso oléfines.

Le procédé de séparation en lit mobile simulé faisant appel au dispositif selon l'invention peut être tel que le fluide principal traversant ledit dispositif a une masse volumique comprise entre 600 et 950 kg/m3 et une viscosité comprise entre 0,1 et 0,6 cPo.

## DESCRIPTION DETAILLEE DE L'INVENTION

Le problème que cherche à résoudre la présente invention est celui de limiter les écarts de temps de séjour dans les zones non sélectives d'un système N-colonnes en série, chaque colonne étant munie d'un lit granulaire. Ces écarts nuisent en effet aux performances de la séparation, tout en minimisant les volumes desdites zones non sélectives (appelés plus simplement dans la suite volumes non sélectifs) qui induisent une augmentation non désirée du débit de « tourne en rond » de l'installation à mêmes niveaux de performance qu'un système dépourvu de zones non sélectives.

L'invention porte sur un système qui permet de garantir une bonne synchronicité du fluide dans le système de distribution/collecte à l'échelle de la section complète de la colonne, et qui réalise par ailleurs un faible volume non sélectif en travaillant de manière adéquate la forme des canaux de distribution et de collecte.

Les colonnes selon l'invention peuvent être organisées selon deux modes d'écoulement :

- Une mise en oeuvre axiale dans laquelle l'écoulement au sein du lit granulaire se fait essentiellement suivant l'axe vertical de la colonne.
- Une mise en oeuvre radiale dans laquelle l'écoulement au sein du lit granulaire se fait essentiellement de la périphérie vers le centre de la colonne ou du centre de la colonne vers la périphérie .

Les dimensions des colonnes sont les suivantes en fonction du mode d'écoulement :

- Pour une mise en oeuvre axiale, un diamètre entre 1 et 15 m, préférentiellement entre 7 et 12 m. La hauteur du lit granulaire varie entre 0,2 et 1,5 m, préférentiellement entre 0,4 et 1m.
- Pour une mise en oeuvre radiale, un diamètre entre 1,5 et 15 m, une hauteur permettant de développer une section comprise entre 1 et 200 m2, préférentiellement entre 5 et 80 m2. La section du lit granulaire évolue en fonction du rayon depuis le collecteur d'entrée jusqu'au collecteur de sortie. L'épaisseur du lit granulaire varie entre 0,2 et 1,5 m, préférentiellement entre 0,4 et 1m.

Les injections et collectes ainsi que les mélanges sont faits en un seul point entre deux colonnes dans le réseau (1).

Selon un premier mode de réalisation représenté sur la figure 1, la mise en oeuvre du lit est axiale, et l'injection dans le lit se fait au moyen d'une conduite (2) sensiblement centrée sur l'axe vertical de la colonne qui alimente par un jet le canal de distribution horizontal (3). Généralement le canal de distribution est équipé d'un brise jet non représenté sur la figure 1. La paroi (4) du canal de distribution (3) a une forme permettant de garantir une vitesse constante $V_{canal}$ dans le canal de distribution (3), ce qui permet de minimiser radicalement les volumes non sélectifs et d'obtenir une répartition homogène du débit dans le lit granulaire (6) .

Cette vitesse est comprise entre 0,1 et 5 m/s, idéalement entre 0,5 et 2,5 m/s. La hauteur du canal (3) définie depuis la grille (5) $h_{\text{sup}}(r)$ à la coordonnée radiale r suit idéalement le profil suivant :

$$h_{\text{sup}}(r) = \frac{1}{2} \frac{V_{SL}}{V_{\text{canal}}} \frac{R^2 - r^2}{r}$$

où $V_{SL}$ est la vitesse superficielle du fluide dans le lit granulaire définie comme le ratio du débit volumique par la section de la colonne et R est le rayon de la colonne.

Le lit granulaire (6) est ensuite alimenté depuis le canal de distribution (3) au travers d'une grille (5).

Le fluide s'écoule au travers du lit granulaire (6) selon une direction sensiblement verticale.

Le fluide est ensuite collecté sous la grille (7) dans le canal de collecte (8).

La paroi (9) du canal de collecte (8) suit idéalement le profil suivant :

$$h_{\text{inf}}(r) = h_{\text{sup}}(r) \frac{r^2}{R^2 - r^2}$$

Ce profil permet de garantir l'égalité des temps de séjour pour l'ensemble des filets fluide entre l'entrée par la conduite (2) et la sortie par la conduite (11).

La représentation des parois 4 et 9 sur la figure 1 sont schématiques et ne correspondent pas à la forme mathématique.

Selon ce mode de réalisation, la collecte du fluide sortant du canal (8) est assurée par un réseau de conduites (10) situé en périphérie, tel que schématisé sur la figure 1.a, qui ramène le fluide sortant dans la conduite d'évacuation (11).

Le nombre de conduites (10) varie généralement entre 4 et 20, préférentiellement entre 6 et 12. Les sections de conduite seront dimensionnées pour assurer une vitesse des fluides comprises entre 1 et 8 m/s.

Par ailleurs on reste dans le cadre de la présente invention en inversant le système de distribution et de collecte, c'est-à-dire plus précisément en utilisant le système de distribution précédemment décrit comme un système de collecte, et en utilisant le système de collecte précédemment décrit comme un système de distribution.

Dans une unité industrielle comportant un enchainement de colonnes, ces colonnes peuvent être toutes avec un système de distribution selon l'invention, ou bien toutes avec un système de collecte selon l'invention. On peut aussi enchainer les colonnes de façon alternée, c'est-à-dire une colonne ayant un système de collecte selon l'invention, et la suivante ayant une système de distribution selon l'invention.

Selon un deuxième mode de réalisation, représenté par la figure 2, la mise en oeuvre du lit est radiale et l'injection dans le lit se fait au moyen d'une conduite verticale (2), ou d'un tube annulaire, positionné sensiblement au centre de la colonne délimité au centre par la paroi (4).

La paroi (4) du canal de distribution (3) a une forme permettant de garantir une vitesse constante $V_{\text{canal}}$ dans le canal de distribution et une répartition homogène du débit dans le lit granulaire (6) . Cette vitesse est comprise entre 0,1 et 5 m/s, idéalement entre 0,5 et 2,5 m/s. La position radiale de la paroi (4) $r_{\text{distrib}}(z)$ comptée depuis l'axe de la colonne, à la coordonnée axiale z comptée depuis l'entrée du fluide dans la colonne suit idéalement le profil suivant :

$$r_{\text{distrib}}(z) = \sqrt{r_{\text{distrib}}(0) - 2 \frac{V_{SL}}{V_{\text{canal}}} r_{\text{distrib}}(0)(H_{\text{lit}} - z)}$$

où $H_{\text{lit}}$ est la hauteur totale du lit granulaire.

Le liquide s'écoule radialement dans le lit (5) depuis le centre vers la périphérie du lit, et est collecté dans les zones périphériques (8). La position radiale de la paroi (9) $r_{\text{collecte}}(z)$ comptée depuis l'axe de la colonne, à la coordonnée axiale z comptée depuis l'entrée du fluide dans la colonne suit idéalement le profil suivant

$$r_{\text{collecte}}(z) = \sqrt{(r_{\text{distrib}}(0) + E_{\text{lit}})^2 + \frac{z}{H_{\text{lit}} - z}(r_{\text{distrib}}(0)^2 - r_{\text{distrib}}(z)^2)}$$

où $E_{\text{lit}}$ est l'épaisseur du lit défini dans le sens radial.

Les représentations données sur la figure (2) sont schématiques et ne reproduisent pas la forme mathématiques de $r_{distrib}(z)$ et $r_{collecte}(z)$.

L'ensemble du débit est collecté par le réseau de conduites (10) situé en périphérie qui ramène le fluide sortant dans la conduite d'évacuation (11).

Soit les colonnes sont toutes avec un système de distribution selon l'invention, soit elles sont toutes avec un système de collecte selon l'invention. On peut aussi enchainer les colonnes de façon alternée, c'est-à-dire une colonne ayant un système de collecte selon l'invention, et la suivante ayant une système de distribution selon l'invention.

On reste dans le cadre de l'invention en inversant le système de distribution et de collecte comme expliqué plus haut, c'est-à-dire plus précisément en utilisant le système de distribution précédemment décrit comme un système de collecte et en utilisant le système de collecte précédemment décrit comme un système de distribution.

L'invention peut également être décrite comme un procédé de séparation en lit mobile simulé faisant appel au dispositif selon l'invention, dans lequel la charge à séparer est un mélange quelconque de composés aromatiques ayant de 7 à 9 atomes de carbone.

L'invention peut encore être vue comme un procédé de séparation en lit mobile simulé faisant appel au dispositif selon l'invention, dans lequel la charge à séparer est un mélange de normales et d'iso paraffines.

L'invention peut encore être vue comme un procédé de séparation en lit mobile simulé faisant appel au dispositif selon l'invention, dans lequel la charge à séparer est un mélange de normales et d'iso oléfines.

L'invention peut encore être vue comme un procédé de séparation en lit mobile simulé faisant appel au dispositif selon l'invention, dans lequel le fluide principal traversant ledit dispositif a une masse volumique comprise entre 600 et 950 kg/m3 une viscosité comprise entre 0,1 et 0,6 cPo.

## EXEMPLES SELON L'ART ANTERIEUR ET SELON L'INVENTION

On considère une colonne de 10 m de diamètre comportant un lit granulaire de 1 m de haut. L'écoulement d'un fluide de densité 725 kg/m$^3$ et de viscosité 0,2 cP se fait à l'intérieur de la colonne avec une vitesse superficielle de 2 cm/s. Cette vitesse superficielle est calculée sur la section de la colonne supposée libre.

L'art antérieur présenté dans le brevet FR2,933,000 explique comment dimensionner une chicane pour redresser la distribution des temps de séjour. Le ratio entre la section annulaire (passage du liquide entre le bord la chicane et la paroi de la colonne) et la section totale de la colonne est choisi identique à celui de l'exemple du brevet, soit 8,3 %. L'homme de l'art se fixera une vitesse maximale dans les canaux de distribution et de collecte et dans les conduites de distributions et de collecte, cette vitesse servira à dimensionner lesdits canaux et lesdites conduites. Dans cet exemple, une vitesse maximale de 2,5 m/s est utilisée. Il en résulte, pour un dimensionnement suivant cet art antérieur un volume non sélectif (distribution et collecte) de 13,2 m$^3$.

Suivant la présente invention, et en prenant une vitesse de dimensionnement des canaux de 1 m/s pour ne pas augmenter la dispersion au sein du lit granulaire, le volume des zones non sélectives est de 2,2 m$^3$.

L'homme de l'art évaluera ajustera éventuellement la vitesse de 1 m/s sur la base d'un raisonnement de perte de charge maximale admissible pour conserver une hauteur équivalente à plateau théorique de 1 mm dans le lit.

La présente invention permet donc de diviser par 2 le volume non sélectif par rapport à l'art antérieur, représenté par le brevet US3,214,247 et pratiquement par 4 le volume non sélectif par rapport à l'art antérieur plus ancien défini par le brevet FR2,933,000.

Ces diminutions de volume sont réalisées en réduisant la dispersion de temps de séjour. Or la réduction du volume non sélectif et de la dispersion, est un élément essentiel dans le maintien des performances de procédé de séparation en lit mobile simulé.

|  | *Volume des zones non sélectives (m$^3$)* | *Variance des zones non sélectives (s$^2$)* |
|---|---|---|
| *Dimensionnement suivant l'art antérieur sans profilage des canaux.* | *26,5* | *4* |
| *Dimensionnement avec profil linéaire (Cf brevet US3, 789, 989)sans compensation.* | *13,2* | *2* |
| *Dimensionnement suivant l'invention avec compensation.* | *7,6* | *1* |

**Revendications**

**1.** Dispositif de distribution ou de collecte d'un fluide au sein d'un lit granulaire (6) contenu dans une colonne destinée à effectuer une séparation en plusieurs composants dans lequel :

- la distribution du fluide entrant se fait au moyen d'un canal de distribution (3) de section variable dans lequel le fluide s'écoule à une vitesse constante $V_{canal}$ comprise entre 0,1 et 5 m/s ;
- la collecte du fluide sortant se fait au moyen d'un canal de collecte (8) de section variable dans lequel le fluide s'écoule à la vitesse constante $V_{canal}$ ;
- dans lequel la colonne est une colonne à écoulement axial,

- la hauteur $h_{sup}$ (r) du canal de distribution (3) définie depuis une première grille (5) disposée entre le lit granulaire (6) et la paroi (4) du canal de distribution (3) en suivant la coordonnée radiale r suit le profil suivant :

$$h_{\text{sup}}(r) = \frac{1}{2} \frac{V_{SL}}{V_{\text{canal}}} \frac{R^2 - r^2}{r}$$

- la hauteur $h_{inf}$ (r) du canal de collecte (8) définie depuis une deuxième grille (7) disposée entre le lit granulaire (6) et la paroi (9) du canal de collecte (8) en suivant la coordonnée radiale r suit le profil suivant :

$$h_{\text{inf}}(r) = h_{\text{sup}}(r) \frac{r^2}{R^2 - r^2}$$

- où $V_{SL}$ est la vitesse superficielle du fluide dans le lit granulaire (6) définie comme le ratio du débit volumique par la section de la colonne, R est le rayon de la colonne ; ou

- dans lequel la colonne est une colonne à écoulement radial,

- la position radiale $r_{distrib}(z)$ de la paroi (4) du canal de distribution (3) comptée depuis l'axe de la colonne en suivant la coordonnée axiale z suit le profil suivant :

$$r_{\text{distrib}}(z) = \sqrt{r_{\text{distrib}}(0) - 2 \frac{V_{SL}}{V_{\text{canal}}} r_{\text{distrib}}(0)(H_{\text{lit}} - z)}$$

- le canal de collecte (8) délimité par la paroi (9) suit un profil radial $r_{collecte}(z)$ comptée depuis l'axe de la colonne en fonction de la coordonnée axiale z suivant la formule :

$$r_{\text{collecte}}(z) = \sqrt{(r_{\text{distrib}}(0) + E_{\text{lit}})^2 + \frac{z}{H_{\text{lit}} - z}(r_{\text{distrib}}(0)^2 - r_{\text{distrib}}(z)^2)}$$

- où $V_{SL}$ est la vitesse superficielle du fluide dans le lit granulaire (6) définie comme le ratio du débit volumique par la section de la colonne, $H_{lit}$ est la hauteur totale de la colonne, z est la coordonnée axiale comptée depuis l'entrée du fluide dans la colonne, $r_{distrib}(0)$ est la position radiale comptée depuis l'axe de la colonne où z est égal à 0, et $E_{lit}$ est l'épaisseur du lit défini dans le sens radial.

2. Procédé en lit mobile simulé utilisant le dispositif de collecte selon la revendication 1, dans lequel la circulation du fluide à l'intérieur du lit de solide granulaire est axiale, et l'injection dans le lit se fait au moyen d'une conduite (2) sensiblement centrée sur l'axe vertical de la colonne, qui alimente le canal de distribution horizontal (3) délimité par la paroi (4) du canal de distribution (3), le lit de solide granulaire (6) étant ensuite alimenté depuis ledit canal de distribution (3) au travers de la première grille (5), et le fluide s'écoule au travers du lit granulaire (6) selon une direction sensiblement verticale, le fluide étant ensuite collecté sous la deuxième grille (7) à partir du canal de collecte (8) délimité par la paroi incurvée (9) du canal de collecte (8) par l'intermédiaire de conduites périphériques (10), et l'ensemble du débit étant rassemblé dans une conduite d'évacuation unique (11) sensiblement centrée selon l'axe vertical de la colonne.

3. Procédé de séparation en lit mobile simulé utilisant le dispositif de collecte selon la revendication 1, dans lequel la circulation des fluides à l'intérieur du lit de solide granulaire se fait de façon radiale, le fluide étant introduit au centre de la colonne par une conduite (2) qui alimente le canal de distribution (3) délimité au centre par la paroi (4) du canal de distribution (3), puis traversant le lit granulaire (6) depuis le centre du lit granulaire (6) délimité par la première grille (5) vers la périphérie du lit granulaire (6) délimitée par la deuxième grille (7) où le fluide est collecté dans le canal de collecte (8) définie à l'extérieur par la paroi (9) du canal de collecte (8), qui ramène le liquide par l'intermédiaire de conduites périphériques (10) vers une conduite d'évacuation (11) sensiblement centrée selon l'axe de la colonne.

4. Procédé de séparation en lit mobile simulé faisant appel au dispositif selon la revendication 1, dans lequel la charge à séparer est un mélange quelconque de composés aromatiques ayant de 7 à 9 atomes de carbone.

5. Procédé de séparation en lit mobile simulé utilisant le dispositif selon la revendication 1, dans lequel la charge à séparer est un mélange de normales et d'iso paraffines.

6. Procédé de séparation en lit mobile simulé utilisant le dispositif selon la revendication 1, dans lequel la charge à séparer est un mélange de normales et d'iso oléfines.

7. Procédé de séparation en lit mobile simulé utilisant le dispositif selon la revendication 1, dans lequel le fluide principal traversant ledit dispositif a une masse volumique comprise entre 600 et 950 kg/m3 une viscosité comprise entre 0,1 et 0,6 cPo.

**Patentansprüche**

1. Vorrichtung zur Verteilung oder zum Sammeln eines Fluids innerhalb eines Granulatbetts (6), das in einer Säule enthalten ist, die dazu bestimmt ist, eine Trennung in mehrere Bestandteile auszuführen, wobei:

- die Verteilung des eintretenden Fluids mittels eines Verteilerkanals (3) mit variablem Querschnitt erfolgt, in dem das Fluid mit einer konstanten Geschwindigkeit $V_{canal}$ zwischen 0,1 und 5 m/s strömt;
- das Sammeln des austretenden Fluids mittels eines Sammelkanals (8) mit variablem Querschnitt erfolgt, in dem das Fluid mit der konstanten Geschwindigkeit $V_{canal}$ strömt;
- wobei die Säule eine Säule mit axialer Strömung ist,

• die von einem ersten Gitter (5), das zwischen dem Granulatbett (6) und der Wand (4) des Verteilerkanals (3) entsprechend der radialen Koordinate r angeordnet ist, definierte Höhe $h_{sup}(r)$ des Verteilerkanals (3) folgt dem folgenden Profil:

$$h_{sup}(r) = \frac{1}{2}\frac{V_{SL}}{V_{canal}}\frac{R^2 - r^2}{r}$$

• die von einem zweiten Gitter (7), das zwischen dem Granulatbett (6) und der Wand (9) des Sammelkanals (8) entsprechend der radialen Koordinate r angeordnet ist, definierte Höhe hinf(r) des Sammelkanals (8) folgt dem folgenden Profil:

$$h_{inf}(r) = h_{sup}(r)\frac{r^2}{R^2 - r^2}$$

• wobei $V_{SL}$ die Oberflächengeschwindigkeit des Fluids im Granulatbett (6) ist, definiert als das Verhältnis des Volumenstroms zum Querschnitt der Säule, R der Radius der Säule ist; oder

- wobei die Säule eine Säule mit radialer Strömung ist,

• die radiale Position $r_{distrib}(z)$ der Wand (4) des Verteilerkanals (3) gerechnet von der Achse der Säule entsprechend der axialen Koordinate z folgt dem folgenden Profil:

$$r_{distrib}(z) = \sqrt{r_{distrib}(0) - 2\frac{V_{SL}}{V_{canal}}r_{distrib}(0)(H_{lit} - z)}$$

• der von der Wand (9) begrenzte Sammelkanal (8) folgt einem radialen Profil $r_{collecte}(z)$ gerechnet von der Achse der Säule abhängig von der axialen Koordinate z gemäß der Formel:

$$r_{collecte}(z) = \sqrt{(r_{distrib}(0) + E_{lit})^2 + \frac{z}{H_{lit} - z}(r_{distrib}(0)^2 - r_{distrib}(z)^2)}$$

• wobei $V_{SL}$ die Oberflächengeschwindigkeit des Fluids im Granulatbett (6) ist, definiert als das Verhältnis des Volumenstroms zum Querschnitt der Säule, $H_{lit}$ die Gesamthöhe der Säule ist, z die axiale Koordinate gerechnet ab dem Eintritt des Fluids in die Säule ist, $r_{distrib}(0)$ die radiale Position gerechnet ab der Achse der Säule ist, wo z gleich 0 ist, und $E_{lit}$ die Dicke des Betts definiert in radialer Richtung ist.

2. Verfahren im simulierten Bewegtbett unter Verwendung der Sammelvorrichtung nach Anspruch 1, wobei die Zirkulation des Fluids im Inneren des Betts von Feststoffgranulat axial erfolgt, und die Injektion in das Bett mittels einer im Wesentlichen auf die senkrechte Achse der Säule zentrierten Leitung (2) erfolgt, die den waagrechten Verteilerkanal (3) speist, der von der Wand (4) des Verteilerkanals (3) begrenzt wird, wobei das Bett von Feststoffgranulat (6) anschließend ausgehend vom Verteilerkanal (3) durch das erste Gitter (5) hindurch gespeist wird, und das Fluid durch das Granulatbett (6) gemäß einer im Wesentlichen senkrechten Richtung strömt, wobei das Fluid anschließend unter dem zweiten Gitter (7) ausgehend vom Sammelkanal (8), der von der gekrümmten Wand (9) des Sammelkanals (8) begrenzt wird, mittels Umfangsleitungen (10) gesammelt wird, und die Gesamtheit der Durchflussmenge in einer einzigen Abfuhrleitung (11) zusammengefasst wird, die im Wesentlichen gemäß der senkrechten Achse der Säule zentriert ist.

3. Trennverfahren im simulierten Bewegtbett unter Verwendung der Sammelvorrichtung nach Anspruch 1, wobei die Zirkulation der Fluide im Inneren des Betts von Feststoffgranulat radial erfolgt, wobei das Fluid in die Mitte der Säule durch eine Leitung (2) eingeführt wird, die den Verteilerkanal (3) speist, der in der Mitte durch die Wand (4) des Verteilerkanals (3) begrenzt wird, dann das Granulatbett (6) ausgehend von der vom ersten Gitter (5) begrenzten Mitte des Granulatbetts (6) zum vom zweiten Gitter (7) begrenzten Umfang des Granulatbetts (6) durchquert, wo das Fluid im Sammelkanal (8) gesammelt wird, der außen durch die Wand (9) des Sammelkanals (8) begrenzt wird, der die Flüssigkeit mittels Umfangsleitungen (10) zu einer Abfuhrleitung (11) zurückführt, die im Wesentlichen gemäß der Achse der Säule zentriert ist.

4. Trennverfahren im simulierten Bewegtbett, das die Vorrichtung nach Anspruch 1 verwendet, wobei die zu trennende Ladung eine beliebige Mischung von aromatischen Verbindungen ist, die 7 bis 9 Kohlenstoffatome haben.

5. Trennverfahren im simulierten Bewegtbett, das die Vorrichtung nach Anspruch 1 verwendet, wobei die zu trennende Ladung eine Mischung von Normal- und Iso-Paraffinen ist.

6. Trennverfahren im simulierten Bewegtbett, das die Vorrichtung nach Anspruch 1 verwendet, wobei die zu trennende Ladung eine Mischung von Normal- und Iso-Olefinen ist.

7. Trennverfahren in einem simulierten Bewegtbett, das die Vorrichtung nach Anspruch 1 verwendet, wobei das die Vorrichtung durchquerende Hauptfluid eine Massendichte zwischen 600 und 950 kg/m$^3$ und eine Viskosität zwischen 0,1 und 0,6 cPo hat.

**Claims**

1. Device for distributing or collecting a fluid within a granular bed (6) contained in a column intended to perform separation into a plurality of components in which:

   - the distribution of the entering fluid is done by means of a distribution channel (3) of variable cross section in which the fluid flows at a constant velocity $V_{canal}$ of between 0.1 and 5 m/s;
- the collection of the exiting fluid is done by means of a collection channel (8) of variable cross section in which the fluid flows at the constant velocity $V_{canal}$;
- wherein the column is a column with axial flow,

• the height $h_{sup}(r)$ of the distribution channel (3) defined from a first grating (5) disposed between the granular bed (6) and the wall (4) of the distribution channel (3), following the radial coordinate r, follows the following profile:

$$h_{sup}(r) = \frac{1}{2}\frac{V_{SL}}{V_{canal}}\frac{R^2 - r^2}{r}$$

• the height $h_{inf}(r)$ of the collection channel (8) defined from a second grating (7) disposed between the granular bed (6) and the wall (9) of the collection channel (8), following the radial coordinate r, follows the following profile:

$$h_{inf}(r) = h_{sup}(r)\frac{r^2}{R^2 - r^2}$$

• where $V_{SL}$ is the surface velocity of the fluid in the granular bed (6) defined as the ratio of the volume flow rate to the cross section of the column, R is the radius of the column; or

- wherein the column is a column with radial flow,

• the radial position $r_{distrib}(z)$ of the wall (4) of the distribution channel (3) counted from the axis of the column, following the axial coordinate z, follows the following profile:

$$r_{distrib}(z) = \sqrt{r_{distrib}(0) - 2\frac{V_{SL}}{V_{canal}}r_{distrib}(0)(H_{lit} - z)}$$

• the collection channel (8) delimited by the wall (9) follows a radial profile $r_{collecte}(z)$ counted from the axis of the column as a function of the axial coordinate z in accordance with the formula:

$$r_{collecte}(z) = \sqrt{(r_{distrib}(0) + E_{lit})^2 + \frac{z}{H_{lit-z}}(r_{distrib}(0)^2 - r_{distrib}(z)^2)}$$

• where $V_{SL}$ is the surface velocity of the fluid in the granular bed (6) defined as the ratio of the volume flow rate to the cross section of the column, $H_{lit}$ is the total height of the column, z is the axial coordinate counted from the entry of the fluid into the column, $r_{distrib}(0)$ is the radial position counted from the axis of the column where z is equal to 0, and $E_{lit}$ is the thickness of the bed defined in the radial direction.

2. Simulated moving bed method using the collection device according to Claim 1, wherein the circulation of the fluid inside the bed of granular solid is axial, and the injection into the bed is done by means of a duct (2) substantially centred on the vertical axis of the column, which supplies the horizontal distribution channel (3) delimited by the wall (4) of the distribution channel (3), the bed of granular solid (6) then being supplied from said distribution channel (3) through the first grating (5), and the fluid flows through the granular bed (6) in a substantially vertical direction, the fluid then being collected beneath the second grating (7) from the collection channel (8) delimited by the curved wall (9) of the collection channel (8) via peripheral ducts (10), and all of the flow being gathered in a single discharge duct (11) substantially centred on the vertical axis of the column.

3. Simulated moving bed separation method using the collection device according to Claim 1, wherein the circulation of the fluids inside the bed of granular solid is done in a radial manner, the fluid being introduced at the centre of the column by a duct (2) that supplies the distribution channel (3) delimited at the centre by the wall (4) of the distribution channel (3), then passing through the granular bed (6) from the centre of the granular bed (6) delimited by the first grating (5) towards the periphery of the granular bed (6) delimited by the second grating (7) where the fluid is collected in the collection channel (8) defined on the outside by the wall (9) of the collection channel (8), which returns the liquid via peripheral ducts (10) towards a discharge duct (11) substantially centred on the axis of the column.

4. Simulated moving bed separation method using the device according to Claim 1, wherein the feedstock to be separated is any mixture of aromatic compounds having from 7 to 9 carbon atoms.

5. Simulated moving bed separation method using the device according to Claim 1, wherein the feedstock to be separated is a mixture of normal and iso-paraffins.

6. Simulated moving bed separation method using the device according to Claim 1, wherein the feedstock to be separated is a mixture of normal and iso-olefins.

7. Simulated moving bed separation method using the device according to Claim 1, wherein the main fluid passing through said device has a density of between 600 and 950 kg/m$^3$ and a viscosity of between 0.1 and 0.6 cPo.

**Figure 1**

(1)

(2)

(3)

(4)

(5)

(6)

(8)

(7)

(9)

(10)

(11)

**Figure 1.a**

(10)

(10)

(10)

Figure 2

## RÉFÉRENCES CITÉES DANS LA DESCRIPTION

**Documents brevets cités dans la description**

- EP 0074815 A
- US 20060108274 A1
- FR 2933000
- US 3214247 A
- US 3789989 A
- WO 9962609 A1
- US 6171502 B1

**Littérature non-brevet citée dans la description**

- **DE SILVA M. ; RODRIGUES A. ; MOTA J.** Effect of dead volumes on the performance of an industrial-scale simulated moving-bed parex unit or p-xylene purification. *l'AIChE Journal,* Janvier 2016, vol. 62 (1